# EUROPEAN PATENT APPLICATION

(11) **EP 1 652 515 A1**
(43) Date of publication of application: **03.05.2006**
(21) Application number: 04771503.2
(22) Date of filing: 04.08.2004
(51) Int. Cl.: A61K 9/16

(54) **PROCESS FOR PRODUCING DRUG ULTRAMICROPARTICLE AND APPARATUS THEREFOR**

(30) Priority: 06.08.2003 JP 2003287943
(71) Applicant: Eisai Co., Ltd., Tokyo 112-8088 (JP)
(72) Inventor: KATO, Akira, (JP); YAMAGUCHI, Takehiro, (JP); NOMURA, Teruko, (JP); ONAI, Katsumi, (JP)
(74) Representative: HOFFMANN EITLE
(86) International application number: PCT/JP2004/011518
(87) International publication number: WO 2005/013938

(57) **Abstract**

The present invention provides fine drug particles with submicron sizes excellent in long-term dispersibility. Specifically, it provides a method for producing ultrafine drug particles having an average particle size of 10 nm to 1000 nm, by 1) dissolving a drug in a good solvent or a mixture of good solvents to prepare a drug-containing solution; 2) mixing the drug-containing solution with a solvent being a poor solvent or a mixture of poor solvents for the drug and being miscible with the drug-containing solution in the good solvent or a mixture of good solvents; and 3) subjecting the prepared mixture directly to emulsification under a set processing pressure using a high-pressure homogenizer without carrying out a pretreatment step for adjusting the drug to have an average particle size of 100 µm or less, and an apparatus for producing the particles.

## Description

### Technical Field

The present invention provides methods and apparatuses for producing ultrafine drug particles excellent in long-term dispersibility.

### Background Art

Ultrafine drug particles with submicron sizes have increasing surface areas with decreasing sizes thereof and thereby enable a markedly increased dissolution rate of an insoluble drug. They serve, for example, to improve the absorption and bioavailability of insoluble drugs, enable intravascular administration of insoluble drugs, and reduce the amount of solubilizers in liquid preparations which may cause various adverse events, and their advantages as above are widely known. In addition, they may advantageously change the pharmacokinetics of a drug after intravenous administration or impart the disposition to a specific organ to a drug by controlling the particle sizes of fine particles or further modifying surfaces of fine particles. Such ultrafine particles may have the possibility of improved retention of a drug at the site of administration upon local administration. They can be applied for improving the content uniformity of a drug in a low-concentration oral preparation.

Such ultrafine drug particles with submicron sizes, however, are difficult to produce. Apart from drugs, techniques for yielding ultrafine particles have been developed in the field of pulverization typically of pigments and silica. In contrast, phenomena such as reaggregation, decomposition, crystallization, and glass transition of drugs prevent production of ultrafine drug particles in the solid state. Specifically, in the production of ultrafine particles, the temperature rises with an increasing energy of pulverization, and this may often invite, for example, decomposition, melting, dissolution in a dispersive medium, or crystal growth due to recrystallization of the drug. Ultrafine particles once prepared are known to undergo particle size growth typically due to reaggregation and/or crystallization.

Known methods for producing ultrafine particles are as follows.

Motoyama et al. disclose a method of pulverizing a drug in the solid state (crystals) by using a wet pulverizer (US Patent No. 4,540,602), in which a solid drug is pulverized in an aqueous solution of a water-soluble polymer to yield fine particles of 500 nm to 5000 nm. Liversidge et al. disclose that a stable dispersion of ultrafine drug particles of less than 400 nm is obtained by allowing the surfaces of fine particles to adsorb a surface modifying agent in an amount of 0.1% to 90% of the weight of the fine particles (US Patent No. 5,145,684).

These methods are suitable for relatively efficiently yielding ultrafine drug particles with submicron sizes, but often invite decomposition, melting or fusing, dissolution in a dispersive medium, and/or crystal growth during or after the production, as described above. In addition, it takes several hours to several days to carry out wet pulverization, and this might invite contamination of products by microorganisms. The dispersive medium wears and thereby contaminates products during pulverization, and this considerably affects the quality of product medicaments. There is also the possibility of contamination of the prepared products by drug particles with a large size.

Consequently, a method of producing ultrafine drug particles as an emulsion by pulverizing a drug in the solid state under a high pressure using a high-pressure homogenizer (high-pressure emulsifier) has been developed and widely used. This method, however, must essentially include a pretreatment step for allowing a solid drug to have particles sizes at a specific level or below (generally 100 µm or less, preferably 25 µm or less) before introducing the solid drug into the homogenizer. This step serves to prevent clogging of the homogenizer, since, when a solid drug is directly introduced into a homogenizer, the homogenizer may often undergo clogging. Consequently, a special device has been developed and ways and means of, for example, gradually increasing the pressure from a low pressure have been carried out. These techniques, however, are still insufficient to completely remove the risk of clogging of channels of homogenizers. In addition, the pulverization using a high-pressure homogenizer is carried out under a very high pressure to yield required energy, but heating may affect the quality. William et al., for example, disclose a method of producing fine drug particles of less than 400 nm by using a Microfluidizer (Microfluidics Inc.) (US Patent No. 5,510,118). This method requires a step comprising about hundred cycles at a pressure of 15000 to 30000 psi and can be applied only to oil-soluble drugs such as cyclosporin.

Another method of producing ultrafine drug particles is a precipitation process by Fessi et al. (Japanese Patent No. 2739896) in which a polymer as a film-forming substance and a drug dissolved in a good solvent are added to a poor solvent. The resulting ultrafine drug particles in a suspension produced by the precipitation process, however, may often have increased particle sizes in a short time in the suspension and may not always keep constant quality of the product easily.

Demands therefore have been made to develop a method and an apparatus for producing ultrafine drug particles with submicron sizes, which minimizes the change in particle size of ultrafine drug particles with time, can produce ultrafine drug particles without the need of a pretreatment step for adjusting the drug to have an average particle size at a predetermined level or less (for example, 100 µm or less), and are easy and convenient. Such a change in particle size may often occur immediately after production.

In addition, demands have been made to develop a method and an apparatus for producing ultrafine drug particles, which can produce ultrafine drug particles at low energy while preventing, for example, a dispersive medium from wearing and thereby contaminating products.

### Disclosure of Invention

The present invention provides a method of producing ultrafine drug particles having an average particle size of 10 nm to 1000 nm, comprising the steps of 1) dissolving a drug in a good solvent or a mixture of good solvents to prepare a drug-containing solution; 2) mixing the drug-containing solution with a solvent being a poor solvent or a mixture of poor solvents for the drug and being miscible with the drug-containing solution in the good solvent or a mixture of good solvents; and 3) subjecting the prepared mixture directly to emulsification under a set processing pressure using a high-pressure homogenizer without carrying out a pretreatment step for adjusting the drug to have an average particle size of 100 µm or less.

According to the present invention, 1) a drug is dissolved in a good solvent or a mixture of good solvents to prepare a drug-containing solution, and 2) the drug-containing solution is mixed with a solvent being a poor solvent or a mixture of poor solvents for the drug and being miscible with the drug-containing solution in the good solvent or a mixture of good solvents. Thereafter, the mixture can be emulsified directly using a high-pressure homogenizer. Alternatively, it is acceptable that the solvent being a poor solvent or a mixture of poor solvents for the drug and being miscible with the drug-containing solution in the good solvent or a mixture of good solvents is circulated in a channel of a high-pressure homogenizer, the drug-containing solution is added to the circulating miscible solvent, and the resulting mixture as intact is directly emulsified using the high-pressure homogenizer. In the present invention, the resulting mixture is preferably immediately emulsified directly using a high-pressure homogenizer after 1) dissolving a drug in a good solvent or a mixture of good solvents to prepare a drug-containing solution, and 2) mixing the drug-containing solution with a solvent being a poor solvent or a mixture of poor solvents for the drug and being miscible with the drug-containing solution in the good solvent or a mixture of good solvents. It is preferred to treat the mixture with the high-pressure homogenizer generally within 5 minutes, preferably within 3 minutes, and more preferably within 1 minute after mixing the drug-containing solution and the miscible solvent.

In the present invention, a dispersing agent may be dissolved in a solvent of at least one of 1) the drug-containing solution in a good solvent or a mixture of good solvents and 2) the solvent being a poor solvent or a mixture of poor solvents for the drug and being miscible with the drug-containing solution in the good solvent or a mixture of good solvents. Specifically, the dispersing agent is preferably dissolved in the solvent 2) being a poor solvent or a mixture of poor solvents for the drug and being miscible with the drug-containing solution in the good solvent or a mixture of good solvents.

The "good solvent or mixture of good solvents" in the present invention is not specifically limited, as long as it can fully dissolve the drug. For example, lower alcohols such as methyl alcohol, ethyl alcohol, n-propyl alcohol or isopropyl alcohol; ketone solvents such as acetone or methyl ethyl ketone; as well as acetonitrile, dioxane, methyl ether, ethyl ether, chloroform, dichloromethane, trichloromethane; and mixtures of these solvents may be proposed.

The "poor solvent or mixture of poor solvents" in the present invention is not specifically limited, as long as it is a solvent or a mixture of good solvents not substantially dissolving the drug and is a solvent miscible with the drug-containing solution of a good solvent or a mixture of good solvents. For examples, water, acidic waters containing a variety of acids, and basic waters containing a variety of bases may be proposed.

The mixing ratio of the drug-containing solution to the poor solvent or mixture of poor solvents is not specifically limited, as long as deposition or precipitation of the drug occurs. The amount of the drug-containing good solvent or mixture of good solvents to be mixed is generally 0.001% to 50% (V/V), preferably 0.01% to 10% (V/V), and more preferably 0.01% to 5% (V/V) to the amount of the poor solvent or mixture of poor solvents.

The concentration of the dispersing agent in the solvent is not specifically limited and is generally 0.01% to 5% (W/V), preferably 0. 1% to 4% (W/V), and more preferably 0.5% to 3% (W/V). The dispersing agent is not specifically limited in its type and can be, for example, any of nonionic surfactants, anionic surfactants, cationic surfactants, amphoteric surfactants, surfactants derived from naturally occurring substances, and hydrophilic polymers. The nonionic surfactants include, but are not limited to, sorbitan fatty acid esters, polyoxyethylene sorbitan fatty acid esters, polyethylene glycol fatty acid esters, sucrose fatty acid esters, polyoxyethylene polyoxypropylene glycol copolymers, polyoxyethylene hydrogenated castor oils, glycerol fatty acid esters, and polyglycerol fatty acid esters. The sorbitan fatty acid esters include sorbitan monostearate, sorbitan sesquioleate, and sorbitan trioleate. The polyoxyethylene sorbitan fatty acid esters are typified by polysorbates 20, 40, 60, and 80. The polyethylene glycol fatty acid esters include polyethylene glycol monolaurate. The sucrose fatty acid esters include sucrose palmitates and sucrose stearic acid. The polyoxyethylene hydrogenated castor oils include polyoxyethylene hydrogenated castor oils 50 and 60. The polyoxyethylene polyoxypropylene glycol copolymers (block copolymers between ethylene oxide and propylene oxide) include polyoxyethylene(160) polyoxypropylene(30) glycol (trade name: Pluronic F-68, BASF AG), polyoxyethylene(196) polyoxypropylene(67) glycol (trade name: Pluronic F-127, BASF AG), and Pluronic L-121 (BASF AG). The glycerol fatty acid esters include glyceryl monostearate (MGS series, Nikko Chemicals Co., Ltd.). The polyglycerol fatty acid esters include tetraglycerol monostearate. The anionic surfactants include sodium glycocholate and sodium deoxycholate. The cationic surfactants include amine salt cationic surfactants and quaternary ammonium salt cationic surfactants. The amphoteric surfactants include amino acid amphoteric surfactants and betaine amphoteric surfactants. The surfactants derived from naturally occurring substances include lecithins such as purified yolk lecithin and hydrogenated soybean lecithin. The hydrophilic polymers include gelatin, polyvinylpyrrolidones, hydroxypropylcellulose, and hydroxypropylmethylcellulose.

Any of the above-mentioned dispersing agents can be used in the present invention. Among them, polyoxyethylene polyoxypropylene glycols, lecithins, gelatin and/or polyvinylpyrrolidone is preferred, of which polyoxyethylene(160) polyoxypropylene(30) glycol (trade name: Pluronic F-68) is more preferred.

The average particle size of fine drug particles produced by the production method according to the present invention is generally 10 to 1000 nm, preferably 50 to 800 nm, and more preferably 100 to 400 nm. The high-pressure homogenizer for use in the present invention is not specifically limited and can be, for example, Microfluidizer (MFIC Corporation, USA), Nanomiser (Yoshida Kikai Co., Ltd.), piston-gap homogenizer, or Manton Gaulin Homogenizer, of which Microfluidizer or Nanomiser is preferred.

The processing pressure in the treatment with a high-pressure homogenizer relating to the present invention is generally 500 to 40000 psi, preferably 1000 to 30000 psi, and more preferably 3000 to 30000 psi.

Specifically, when the high-pressure homogenizer is Microfluidizer, the processing pressure is generally 1000 to 20000 psi, preferably 1000 to 10000 psi, and more preferably 3000 to 6000 psi. When the high-pressure homogenizer is Nanomiser, the processing pressure is generally 1000 to 40000 psi, preferably 5000 to 30000 psi, and more preferably 6000 to 20000 psi.

The temperature of solvent in the treatment with a high-pressure homogenizer relating to the present invention can be low temperatures and is not specifically limited, as long as the solvent is neither solidified nor becomes viscous. It is generally preferably 40°C or lower.

The present invention also provides a method of producing a suspension of ultrafine drug particles or powdered ultrafine drug particles in an arbitrary concentration, the ultrafine drug particles having an average particle size of 10 nm to 1000 nm, which comprises the steps of 1) dissolving a drug in a good solvent or a mixture of good solvents to prepare a drug-containing solution; 2) mixing the drug-containing solution with a solvent being a poor solvent or a mixture of poor solvents for the drug and being miscible with the drug-containing solution in the good solvent or a mixture of good solvents; 3) subjecting the prepared mixture directly to emulsification under a set processing pressure using a high-pressure homogenizer without carrying out a pretreatment step for adjusting the drug to have an average particle size of 100 µm or less; and 4) removing part or all of the solvent from the suspension of ultrafine drug particles after the treatment with the high-pressure homogenizer.

The step of removing part or all of the solvent from the suspension of ultrafine drug particles after the treatment with the high-pressure homogenizer can be carried out by any procedure such as freeze-drying, circulation drying, vacuum drying, or drying under reduced pressure. This step is performed in order to increase the drug concentration in the suspension of ultrafine drug particles and/or to recover solid or powdery ultrafine drug particles and can be used to secondary processing for formulating the particles into dosage forms such as injections, powders, granules, tablets, capsules, ointments, percutaneous agents, suppositories, drinkable preparations, syrups, and liquid preparations.

The drug for use in the present invention is not specifically limited in its type and includes antitumor drugs, antibiotics, anti-inflammatory drugs, analgesics, drugs for treating osteoporosis, hypolipidemic drugs, antibacterial drugs, sedative drugs, tranquilizers, antiepileptic drugs, antidepressants, drugs for treating digestive system diseases, drugs for treating allergic diseases, antihypertensive drugs, antiarteriosclerosis drugs, antidiabetic drugs, hormone drugs, and lipid soluble vitamin preparations. The antitumor drugs include N-(3-chloro-7-indolyl)-1,4-benzenedisulfonamide, danazol, piposulfam, camptothecin, triiodobenzoate, taxol, doxorubicin hydrochloride, methotrexate, etoposide, 5-fluorouracil, mitoxantrone, mesna, dimesna, aminoglutethimide, tamoxifen, acroline, cisplatinum, carboplatin, and cyclophosphamide. The antibiotics include amikacin and gentamicin. Examples of the anti-inflammatory drugs are aspirin, phenacetin, acetaminophenone, phenylbutazone, ketophenylbutazone, mefenamic acid, bucolome, benzydamine, mepirizole, tiaramide, tinoridine, anti-inflammatory steroids such as prednisolone, hydrocortisone, methylprednisolone, dexamethasone, and betamethasone, as well as indomethacin, diclofenac, loxoprofen, ibuprofen, and piroxicam. The analgesics include Xylocaine, pentazocine, and aspirin. The drugs for treating osteoporosis include vitamin K2, prostaglandin A1, vitamin D, sexual hormone derivatives, phenolsulfophthalein, benzothiopyran, and thienoindazole. The antihyperlipemic drugs include clinofibrate, clofibrate, cholestyramine, soysterol, tocopherol nicotinate, nicomol, niceritrol, probucol, and elastase. The tranquilizers include benzodiazepines such as diazepam, lorazepam, and oxazolam. The antiepileptic drugs include phenytoin, phenobarbital, carbamazepine, and primidone. The antidepressants include imipramine, noxiptiline, and phenelzine. The drugs for treating digestive system diseases include metoclopramide, famotidine, omeprazole, sulpiride, and trepibutone. The drugs for treating allergic diseases include clemastine fumarate, cyproheptadine hydrochloride, diphenhydramine, methdilazine, clemizole, and methoxyphenamine. The antihypertensive drugs include nicardipine hydrochloride, delapril hydrochloride, captopril, prazosin hydrochloride, and reserpine. The antiarteriosclerosis drugs include drugs for inhibiting cholesterol ester transfer protein. The antidiabetic drugs include glymidine, glipzide, glibenclamide, buformin, and metformin. The hormone drugs include dexamethasone, betamethasone, prednisolone, hydrocortisone, triamcinolone, triamcinolone acetonide, fluocinolone acetonide, hexestrol, methimazole, and estriol. The lipid-soluble vitamin preparations include vitamin A, vitamin D, vitamin E, vitamin K, and folic acid. Specifically preferred drugs for use in the production of fine drug particles according to the present invention are insoluble drugs having a solubility in water of 1 mg/ml or less and include, but are not limited to, N-(3-chloro-7-indolyl)-1,4-benzenedisulfonamide known as an antitumor drug for inhibiting the growth of cancer cells. N-(3-Chloro-7-indolyl)-1,4-benzenedisulfonamide by itself is insoluble or slightly soluble under substantially physiological conditions, i.e., at pH 5 to 7.

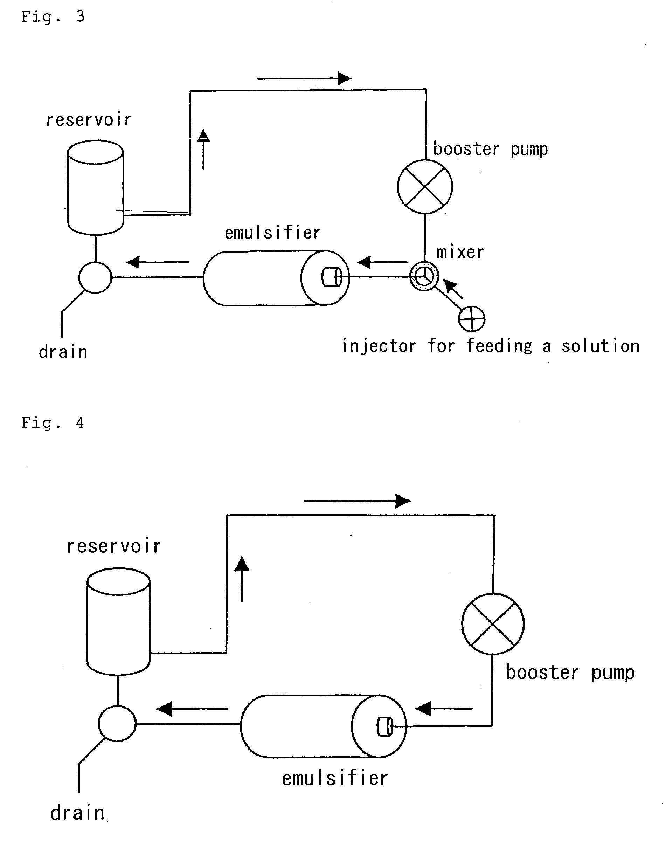

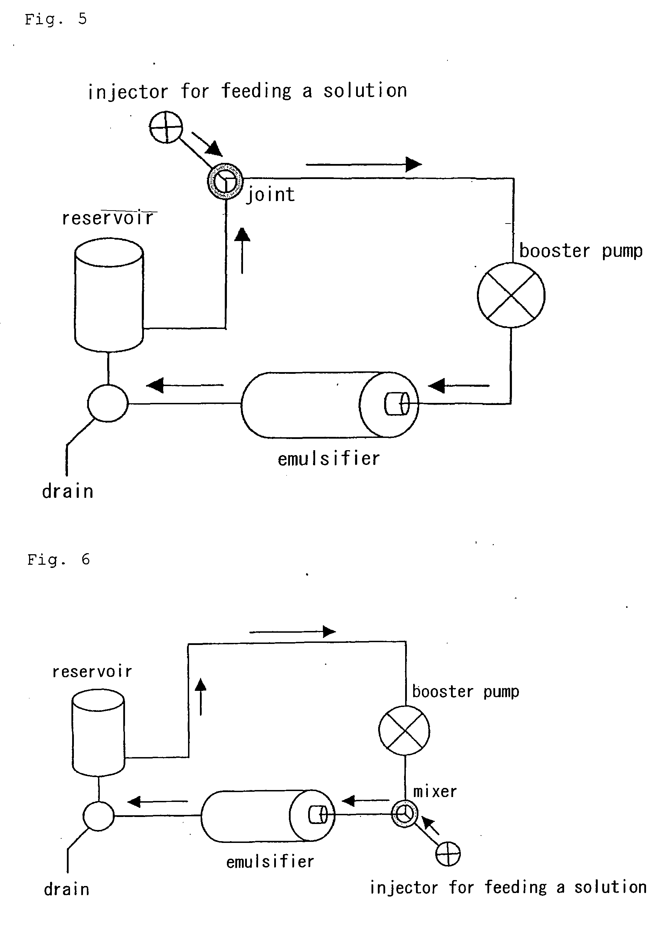

The present invention further provides a high-pressure homogenizer equipped with an online injector comprising a high-pressure homogenizer and an injector, the high-pressure homogenizer shown in the following Fig. 4, comprising a reservoir, a booster pump, and an emulsifier being connected via thin tubes, wherein the injector is so configured as to feed a drug-containing solution containing a drug dissolved in a good solvent or a mixture of good solvents and wherein the injector is integrated to the high-pressure homogenizer at any position of a channel for a circulating fluid in the thin tubes extending from the reservoir to the emulsifier. The injector can be integrated at any position of a channel in the thin tube connecting between the reservoir and the booster pump as shown in the following Fig. 5 or can be integrated at any position of a channel in the thin tube connecting between the booster pump and the emulsifier as shown in the following Fig. 6. The injector can be integrated at any position of a channel in the thin tubes with the interposition of a joint and/or a mixer. The present invention provides a high-pressure homogenizer equipped with an online injector which can feed the drug-containing solution of a good solvent or a mixture of good solvents through the injector immediately before emulsification in the high-pressure homogenizer.

The high-pressure homogenizer equipped with an online injector according to the present invention may further comprise a regulator serving to regulate the temperature of the circulating fluid and/or the drug-containing solution and being integrated into part or all of (1) the emulsifier and/or (2) the thin tubes connecting among the reservoir, the pressure pump and the emulsifier. The liquid-temperature regulator is not specifically limited, as long as it is a device that can regulate the temperature of the circulating fluid and/or the drug-containing solution. It is preferred that the regulator can serve to remove the heat liberated typically from the emulsifier. A device for circulating cooling water around the emulsifier, for example, can be integrated.

The high-pressure homogenizers equipped with an online injector can be used as a high-pressure homogenizers for use in the production methods of ultrafine drug particles according to the present invention. Specifically, they can be used in the method of producing ultrafine drug particles having an average particle size of 10 nm to 1000 nm, comprising the steps of 1) dissolving a drug in a good solvent or a mixture of good solvents to prepare a drug-containing solution; 2) mixing the drug-containing solution with a solvent being a poor solvent or a mixture of poor solvents for the drug and being miscible with the drug-containing solution in the good solvent or a mixture of good solvents; and 3) subjecting the prepared mixture directly to emulsification under a set processing pressure using a high-pressure homogenizer without carrying out a pretreatment step for adjusting the drug to have an average particle size of 100 µm or less and/or the method of producing powdered ultrafine drug particles or a suspension of ultrafine drug particles in an arbitrary concentration, the ultrafine drug particles having an average particle size of 10 nm to 1000 nm, comprising the steps of 1) dissolving a drug in a good solvent or a mixture of good solvents to prepare a drug-containing solution; 2) mixing the drug-containing solution with a solvent being a poor solvent or a mixture of poor solvents for the drug and being miscible with the drug-containing solution in the good solvent or a mixture of good solvents; 3) subjecting the prepared mixture directly to emulsification under a set processing pressure using a high-pressure homogenizer without carrying out a pretreatment step for adjusting the drug to have an average particle size of 100 µm or less; and 4) removing part or all of the solvent from the suspension of ultrafine drug particles after the treatment with the high-pressure homogenizer. More specifically, it is possible that the solvent being a poor solvent or a mixture of poor solvents for the drug and being miscible with the drug-containing solution is circulated through a channel in the high-pressure homogenizer equipped with an online injector, and the drug-containing solution is fed through the injector to the circulating miscible solvent to thereby mix them, and the resulting mixture is directly emulsified online at a set processing pressure using the high-pressure homogenizer. The high-pressure homogenizers equipped with an online injector according to the present invention can produce ultrafine drug particles having an average particle size of 10 nm to 1000 nm in an arbitrary concentration.

The methods according to the present invention produce ultrafine drug particles by 1) dissolving a drug in a good solvent or a mixture of good solvents to prepare a drug-containing solution, 2) mixing the drug-containing solution with a solvent being a poor solvent or a mixture of poor solvents for the drug and being miscible with the drug-containing solution in the good solvent or a mixture of good solvents; and 3) subjecting the prepared mixture to treatment with a high-pressure homogenizer. Thus, the methods have a surprising feature of producing ultrafine drug particles excellent in long-term dispersibility by preventing precipitation and aggregation of drug particles. Methods of producing fine drug particles have been already known in the conventional technologies. The fine drug particles in a solvent produced by the production methods according to the present invention, however, can have particle sizes held stably without substantial increase with time, in contrast to the conventional technologies. This is a very advantageous and distinguished feature in storage and secondary processing after production of fine drug particles.

Another feature of the production methods according to the present invention is that the methods do not require a pretreatment step for adjusting the drug to have an average particle size at a predetermined level or less (generally 100 µm or less and preferably 25 µm or less) in the production of ultrafine drug particles using a high-pressure homogenizer (high-pressure emulsifier) and can easily and conveniently carry out the treatment with a high-pressure homogenizer. Specifically, if a solid drug is directly introduced into a high-pressure homogenizer for use in the conventional methods of producing ultrafine drug particles by treating drug particles as intact with the high-pressure homogenizer (high-pressure emulsifier), the channel of the high-pressure homogenizer is often clogged. To avoid such clogging of the channel in the homogenizer, the conventional methods must essentially include a pretreatment step of pulverizing the drug particles to such diameters as not to clog a channel of the high-pressure homogenizer. More specifically, they require a pretreatment step of achieving the average particle size of 100 µm or less as 90% particle size, and this constitutes a significant limitation in production of ultrafine drug particles. The present invention, however, does not require such a pretreatment step and can very easily and conveniently produce ultrafine drug particles by direct treatment with a high-pressure homogenizer at a set pressure.

Yet another surprising feature of the production methods according to the present invention is that ultrafine drug particles can be produced by using a high-pressure homogenizer even at a low energy output. Specifically, regarding the energy output of an emulsifier constituting a high-pressure homogenizer, the processing pressure in the treatment with a high-pressure homogenizer according to the present invention is lower than those in conventional methods. More specifically, the processing pressure in treatment with a high-pressure homogenizer according to the conventional methods is generally 14000 psi to 60000 psi. In contrast, the processing pressure in the present invention is generally 500 to 40000 psi, preferably 1000 to 30000 psi, and more preferably 3000 to 30000 psi. In addition, the treatment with a high-pressure homogenizer can be carried out at lower temperatures of the solvent. These are astonishing features as compared with the conventional methods.

The high-pressure homogenizer equipped with an online injector according to the present invention can significantly advantageously produce ultrafine drug particles excellent in long-term dispersibility efficiently and stably when used in the production methods of ultrafine drug particles according to the present invention. This apparatus serves to easily and conveniently produce fine drug particles having a stable average particle size in a solvent substantially without an increase with time. The high-pressure homogenizer equipped with an online injector according to the present invention enables direct emulsification at a set processing pressure without carrying out a pretreatment step for adjusting the drug to have an average particle size at a predetermined level or less, when used in the methods of producing ultrafine drug particles according to the present invention. This apparatus enables production of ultrafine drug particles by treatment with the high-pressure homogenizer at a low energy output, i.e., at low pressures and low temperatures. In addition, by using the high-pressure homogenizer equipped with an online injector according to the present invention, two solutions can be mixed immediately before emulsification, and thereby the apparatus enables mixing and emulsification of two or more solutions which are inherently difficult to mix with each other.

The present invention also provides use of the high-pressure homogenizer equipped with an online injector for emulsification of a drug-containing solution.

The ultrafine drug particles relating to the present invention can be produced, for example, by the following method.

For example, 1 g of Pluronic F68 is dissolved in purified water to yield 100 g of an aqueous solution (1% aqueous solution of Pluronic F68), and 1.7 mL of the aqueous solution is circulated in a Microfluidizer as a high-pressure homogenizer. Separately, 5 g of an insoluble drug (e.g., N-(3-chloro-7-indolyl)-1,4-benzenedisulfonamide) is dissolved in acetone to yield 100 g of a 50 mg/mL solution of N-(3-chloro-7-indolyl)-1,4-benzenedisulfonamide) in acetone. To the circulating 1% aqueous solution of Pluronic F68 is added 100 µL of the 50 mg/mL solution of N-(3-chloro-7-indolyl)-1,4-benzenedisulfonamide) in acetone, and the mixture is subjected to treatment with a high-pressure homogenizer (e.g., pressurization at 3000 psi for 10 minutes using the Microfluidizer). Thus, ultrafine drug particles can be prepared. Powdery fine drug particles can be obtained by subjecting the resulting suspension of ultrafine drug particles to solvent removing process such as freeze-drying or spray drying.

When a high-pressure homogenizer comprising a reservoir, a booster pump, and an emulsifier connected through thin tubes can be configured into a high-pressure homogenizer equipped with an online injector by integrating a sample injector for feeding a drug-containing solution of a good solvent or a mixture of good solvents into at any portion in the thin tubes from the reservoir to the emulsifier through which a circulating fluid passes, the resulting apparatus enables efficient production of fine drug particles which are stable over a long time.

According to the present invention, ultrafine drug particles which are excellent in long-term dispersibility and are substantially free from change in particle size with time can be produced.

### Brief Description of Drawings

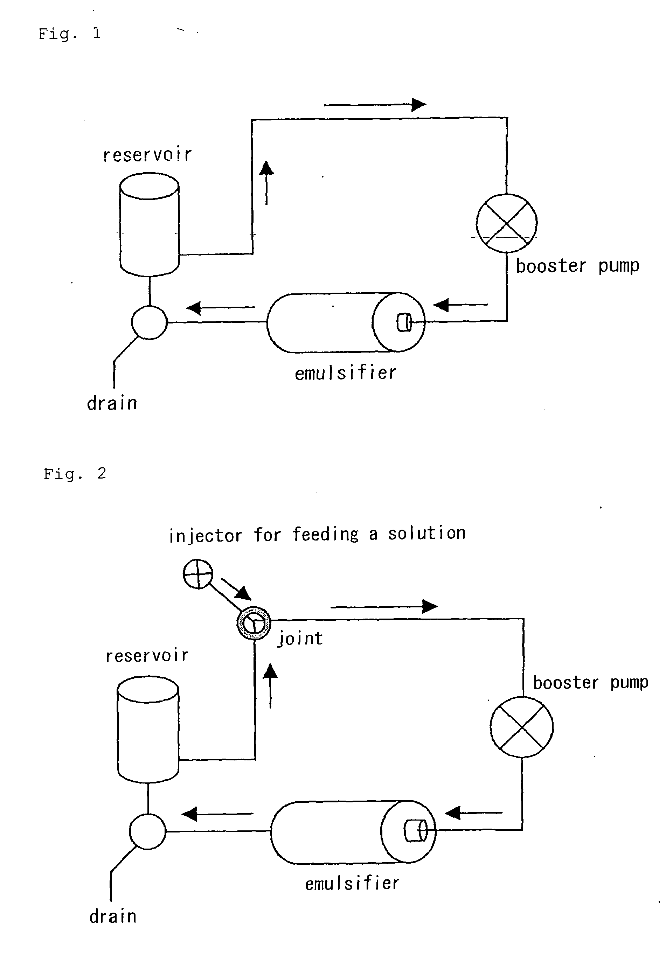

Fig. 1 is a schematic view of a high-pressure homogenizer comprising a reservoir, a booster pump, and an emulsifier connected via thin tubes.
Fig. 2 is a schematic view of a high-pressure homogenizer equipped with an online injector, having an injector integrated at any position in a channel of a thin tube connecting between the booster pump and the emulsifier, the injector serving to feed a solution of a drug in a good solvent or a mixture of good solvents.
Fig. 3 is a schematic view of a high-pressure homogenizer equipped with an online injector, having an injector integrated at any position in a channel of a thin tube connecting between the reservoir and the booster pump, the injector serving to feed a solution of a drug in a good solvent or a mixture of good solvents.
Fig. 4 is a schematic view of a high-pressure homogenizer comprising a reservoir, a booster pump, and an emulsifier connected via thin tubes.
Fig. 2 is a schematic view of a high-pressure homogenizer equipped with an online injector, having an injector integrated at any position in a channel of a thin tube connecting between the booster pump and the emulsifier, the injector serving to feed a solution of a drug in a good solvent or a mixture of good solvents.
Fig. 6 is a schematic view of a high-pressure homogenizer equipped with an online injector, having an injector integrated at any position in a channel of a thin tube connecting between the reservoir and the booster pump, the injector serving to feed a solution of a drug in a good solvent or a mixture of good solvents.

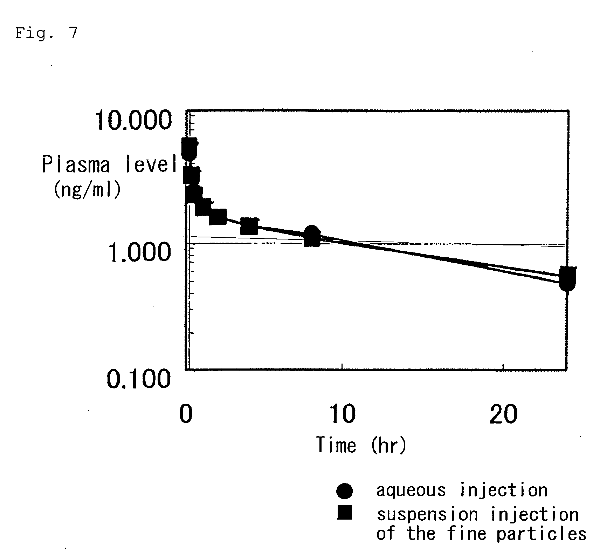

Fig. 7 is plasma level profiles until 24 hours after the administration, in which an injection of suspended fine particles of the N-(3-chloro-7-indolyl)-1,4-benzenedisulfonamide obtained according to Example 1 and an aqueous solution of the drug as a comparative example were intravenously administered to rats (n=4) at a dosage of 0.5 mg/kg per individual.

1) Effects of the "method of producing fine drug particles" according to the present invention on average particle size of fine drug particles and stability thereof:

### (A) Comparison with various emulsification/pulverization devices

A suspension of fine drug particles was prepared by circulating 7 mL of an aqueous solution containing 1% Pluronic F68 through a Microfluidizer, adding 100 µL of a solution of N-(3-chloro-7-indolyl)-1,4-benzenedisulfonamide (50 mg/mL) in acetone to the circulating solution, and applying a pressure at 3000 psi for 20 minutes, as illustrated in the following Example 1.

Separately, fine drug particles were prepared according to Comparative Examples by direct emulsification using high-pressure emulsification (Comparative Example 1: Microfluidizer treatment, Comparative Example 2: piston-gap homogenizer treatment, and Comparative Example 3: Nanomiser treatment), wet pulverization (Comparative Example 4: Dyno-Mill treatment), or ultrasonic process (Comparative Example 5: sonicator treatment) according to the following methods. In Comparative Example 1 (Microfluidizer treatment), Comparative Example 2 (piston-gap homogenizer treatment), and Comparative Example 3 (Nanomiser treatment), N-(3-chloro-7-indolyl)-1,4-benzenedisulfonamide to be used had been subjected to a pretreatment step so as to have an average particle size of 100 µm or less, in which the drug had been treated once using a jet mill.

### (Comparative Examples 1 to 5)

### Comparative Example 1: Microfluidizer treatment

A suspension of fine drug particles was prepared by dispersing and suspending, to a concentration of 50 mg/mL, N-(3-chloro-7-indolyl)-1,4-benzenedisulfonamide previously treated to have an average particle size of 100 µm or less in 7 mL of a 1% aqueous solution of Pluronic F68; circulating the suspension through a Microfluidizer; and pressurizing the circulating suspension at 6000 psi for 10 minutes.

### Comparative Example 2: Piston-gap homogenizer treatment

A suspension of fine drug particles was prepared by dispersing and suspending, to a concentration of 50 mg/mL, N-(3-chloro-7-indolyl)-1,4-benzenedisulfonamide previously treated to have an average particle size of 100 µm or less in 50 mL of a 0.5% aqueous solution of methylcellulose; circulating the suspension through a piston-gap homogenizer; and pressurizing the circulating suspension at 14500 psi for 10 minutes.

### Comparative Example 3: Nanomiser treatment

A suspension of fine drug particles was prepared by dispersing and suspending, to a concentration of 50 mg/mL, N-(3-chloro-7-indolyl)-1,4-benzenedisulfonamide previously treated to have an average particle size of 100 µm or less in 7 mL of a 1% aqueous solution of Pluronic F68; circulating the suspension through a Nanomiser; and pressurizing the circulating suspension at 15000 psi for 10 minutes.

### Comparative Example 4: Dyno-Mill treatment

A suspension of fine drug particles was prepared by dispersing and suspending N-(3-chloro-7-indolyl)-1,4-benzenedisulfonamide to a concentration of 50 mg/mL in 500 mL of a 0.5% aqueous solution of methylcellulose; and treating the suspension with a Dyno-Mill for 10 minutes.

### Comparative Example 5: Sonicator treatment

A suspension of fine drug particles was prepared by dispersing and suspending N-(3-chloro-7-indolyl)-1,4-benzenedisulfonamide to a concentration of 50 mg/mL in 500 mL of a 0.5% aqueous solution of methylcellulose; and treating the suspension with an ultrasonic disperser.

To compare the fine drug particles prepared in Example 1 with those prepared in Comparative Examples 1 to 5, the average particle sizes of these particles were determined using a light scattering photometer Model DLS-7000 DL (a product of Otsuka Electronics Co., Ltd.) (measuring conditions: measuring temperature: 25°C, number of accumulations: 200 times). Values determined by the cumulant method were defined as the average particle sizes.

The determined average particle sizes are shown in Table 1.

**Table 1**

| | Preparation Method | Apparatus | Average particle size (nm) |
|---|---|---|---|
| Example 1 | the present invention | Microfluidizer | 218 |
| Comparative Example 1 | high-pressure emulsification | Microfluidizer | 856 |
| Comparative Example 2 | high-pressure emulsification | piston-gap homogenizer | 387 |
| Comparative Example 3 | high-pressure emulsification | Nanomizer | 366 |
| Comparative Example 4 | wet pulverization | Dyno-Mill | 465 |
| Comparative Example 5 | ultrasonication | sonicator | 490 |

### (B) Comparison with stirring process in a method of adding a drug-containing solution of a good solvent to a poor solvent

A suspension of fine drug particles was prepared as shown in the following Example 2 by circulating 7 mL of a 1% aqueous solution of Pluronic F68 through a Microfluidizer; adding 100 µL of a 50 mg/mL solution of N-(3-chloro-7-indolyl)-1,4-benzenedisulfonamide in acetone to the circulating solution; and pressurizing the mixture at 3000 psi for 20 minutes. The average particle size of fine drug particles in the resulting suspension was sequentially determined 1, 2, 4, 6, 8, 24 and 48 hours later.

Separately, a suspension of fine drug particles was prepared as Comparative Example 6 by placing 7 mL of a 1% aqueous solution of Pluronic F68 in a beaker; adding 100 µL of a 50 mg/mL solution of N-(3-chloro-7-indolyl)-1,4-benzenedisulfonamide in acetone to the aqueous solution with stirring using a magnetic stirrer (stirrer bar); and stirring the mixture for further 5 minutes. The change of the average particle size of drug particles in the resulting suspension was sequentially determined 3, 7, 10, 13, 16, 27, 36, 59, 94, and 122 hours later.

The determined changes in average particle size with time are shown in Table 2.

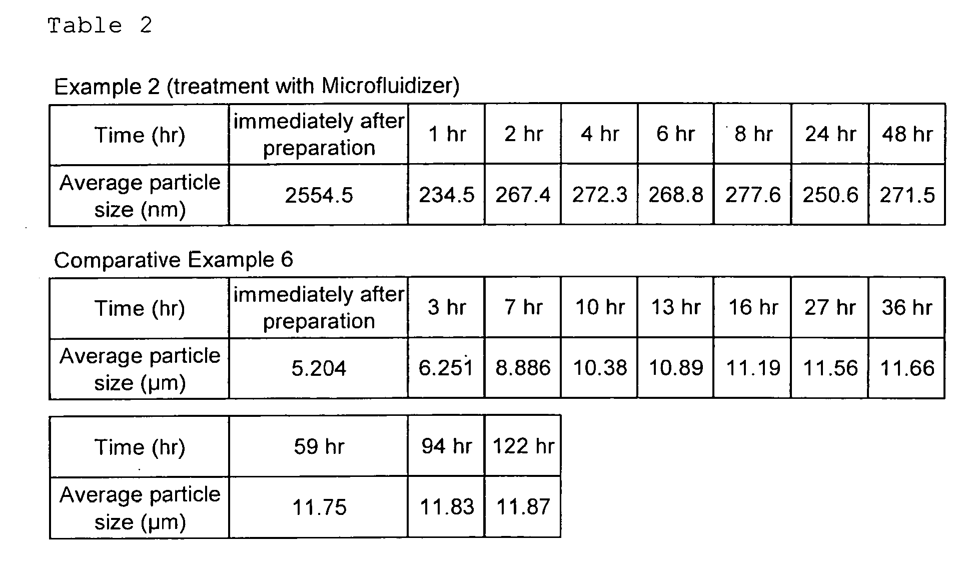

Table 1 demonstrates that the fine drug particles of N-(3-chloro-7-indolyl)-1,4-benzenedisulfonamide can be produced according to any of the methods according to the present invention (Example 1) and Comparative Examples 1 to 5 (direct emulsification using high-pressure emulsification, wet pulverization, and ultrasonic process), but that ultrafine drug particles having the minimum average particle size can be obtained by the "method of producing ultrafine drug particles comprising, which comprises circulating a poor solvent in a high-pressure homogenizer; adding a drug-containing solution of good solvent to the circulating poor solvent; and carrying out pressurization and emulsification" according to the present invention. It also shows that ultrafine drug particles having a smaller average particle size can be prepared according to the present invention without conducting a pretreatment step for adjusting the drug to have an average particle size of 100 µm or less, as compared with Comparative Examples 1 to 3 in which the pretreatment step was carried out.

Further, Table 2 shows that, of fine drug particles of N-(3-chloro-7-indolyl)-1,4-benzenedisulfonamide prepared by the "method of adding a drug-containing solution in a good solvent to a poor solvent", those prepared by treatment with a high-pressure homogenizer (Example 2) have an average particle size of 254.5 nm immediately after preparation, which is significantly finer than that (5.204 µm) of particles prepared in Comparative Example 6 by the stirring process. Relating to stability with time, the ultrafine particles prepared by the treatment with a high-pressure homogenizer do not show a significant change in average particle size even after 48 hours (48 hours later: 271.5 nm). In contrast, the particles prepared by the stirring process show a remarkable increase in particle size. The average particle size 36 hours later is 11.66 µm, about 2 times or more the average particle size immediately after preparation.

These results clearly show that fine drug particles which are excellent in long-term dispersibility and have stable particle sizes substantially free from a significant increase can be obtained according to the present invention, and that the production method according to the present invention is an easy and convenient production method that can directly produce ultrafine drug particles at a set processing pressure using a high-pressure homogenizer without the need of a pretreatment step for adjusting the drug to have an average particle size of 100 µm or less. 2) Influence of the processing pressure of the high-pressure homogenizer in the "method of producing fine drug particles" according to the present invention

As is shown in the following Examples 3 to 5, 100 µL of a 50 mg/mL solution of N-(3-chloro-7-indolyl)-1,4-benzenedisulfonamide in acetone was added to 7 mL of a 1% aqueous solution of Pluronic F68, and the resulting suspension was circulated through a Nanomiser, and a pressure was applied at 8700 psi (Example 3), 15600 psi (Example 4), or 20900 psi (Example 5) for 20 minutes. As a result, fine drug particles having an average particle size of about 200 nm were obtained by any of the treatments at relatively low pressures, as shown in Table 3.

These results demonstrate that the treatments with a high-pressure homogenizer according to the present invention can produce stable fine drug particles under relatively low pressures.

**Table 3**

| | Processing pressure | Average particle size (nm) |
|---|---|---|
| Example 3 | 0.2 MPa | 172.7 |
| Example 4 | 0.3 MPa | 178.8 |
| Example 5 | 0.4 MPa | 211.3 |

### 3) Influence of the solvent temperature of the treatment with a high-pressure homogenizer in the "method of producing fine drug particles" according to the present invention

As is shown in the following Examples 6 and 7, 7 mL of a 1% aqueous solution of Pluronic F68 was circulated through a Nanomiser, whose temperature in the emulsifier section was held to 5°C (Example 6) or 20°C (Example 7) using a constant-temperature water bath, 100 µL of a 50 mg/mL solution of N-(3-chloro-7-indolyl)-1,4-benzenedisulfonamide in acetone was added to the circulating solution, and a pressure at about 19000 psi was applied. Changes in temperature of the sample after treating with the Nanomiser 1 to 50 pass, and changes in clarity and color of solution and in average particle size of the sample upon production after 50-pass treatment and after standing to cool for one hour were determined. The results are shown in Table 4. The "50-pass treatment" means that the sample was treated with the Nanomiser, the recycled mixture was retreated with the Nanomiser immediately, and thus Nanomiser treatment was repeated a total of 50 times.

**Table 4**

| | Temperature in the emulsifier section | Temperature of the sample (°C) | | | | |
|---|---|---|---|---|---|---|
| | | 1 pass | 5 pass | 10 pass | 20 pass | 50pass |
| Example 6 | 5°C | 34°C | 41 °C | 45°C | 47°C | 47°C |
| Example 7 | 20°C | 43°C | 47°C | 49°C | 52°C | 51 °C |
| Comparative Example 7 | not controled | 44°C | 58°C | 67°C | 75°C | 78°C |
| | | | | | | |
| | Clarity and color of the sample (change in particle size) | | | | | |
| | the sample upon production after 50-pass treatment | after standing to cool | | | | |
| Example 6 | white turbid solution of the fine drug particles | white turbid solution of the fine drug particles (the average particle size was not changed) | | | | |
| Example 7 | white turbid solution of the fine drug particles | white turbid solution of the fine drug particles (the average particle size was not changed) | | | | |
| Comparative Example 7 | transparent solution | large crystals were precipitated (the average particle size was increased) | | | | |

When the temperature of the emulsifier section was controlled in Nanomiser treatment according to Examples 6 and 7, the temperature of the sample (temperature of the drug-containing solvent) was elevated from about 30°C to about 50°C with elapse of time, but the clarity and color of the produced fine drug particles were stable without any change even after standing to cool for one hour.

In contrast, when the temperature of the emulsifier section was not controlled in Nanomiser treatment according to Comparative Example 7, N-(3-chloro-7-indolyl)-1,4-benzenedisulfonamide was once dissolved, and standing to cool resulted in deposition of crystals and increase in average particle size.

These results show that the treatment with a high-pressure homogenizer at such a low temperature as not to dissolve the drug can yield stable fine drug particles.

### 4) Effects of fine drug particles prepared by the "method of producing fine drug particles" according to the present invention on bioabsorptivity

The fine particles of N-(3-chloro-7-indolyl)-1,4-benzenedisulfonamide prepared according to Example 1 were intravenously administered to rats (n=4) at a dosage of 0.5 mg/kg under the following conditions, and the blood level of the drug was determined until 24 hours after the administration. As a control test, an aqueous injection of N-(3-chloro-7-indolyl)-1,4-benzenedisulfonamide prepared by the following method was administered to rats by the same procedure as above, and the determination was performed. The results are shown in Fig. 7.

### A) Method of producing an aqueous injection of N-(3-chloro-7-indolyl)-1,4-benzenedisulfonamide:

In a beaker was placed 7 mL of a 1% aqueous solution of Pluronic F68. While stirring, 100 µL of a 50 mg/mL solution of N-(3-chloro-7-indolyl)-1,4-benzenedisulfonamide in acetone was added thereto and the mixture was stirred for further 15 seconds. The resulting suspension was circulated through a Nanomiser whose temperature in the emulsifier section was controlled to 5°C, and a pressure at 10600 psi was applied for about 30 minutes to thereby yield a suspension of ultrafine drug particles. The suspension was diluted with distilled water for injection to a concentration of 0.5 mg/mL, was adjusted with mannitol to be isotonic, and thereby yielded an aqueous injection.

### B) Method and conditions in administration to rats:

The aqueous injection was administered to the caudal vein of SD male rats of 23 weeks old at a dosage of 1 mg/kg (0.5 mg/mL). The blood was sampled from the posterior cervical vein 5, 15, 30 minutes, 1, 2, 4, 8, and 24 hours into the administration, and the plasma was separated therefrom.

### C) Methods of determining the blood level, of extracting and pretreating, and HPLC conditions:

An internal standard substance and methanol were mixed with 50 µL of the collected plasma, respectively. The resulting mixture was further mixed with water and ether, was vigorously stirred, was centrifuged, and the supernatant was collected. The residual aqueous layer was mixed another portion of ether and was subjected to the same procedure as above. The supernatants were collected and were evaporated to dryness under nitrogen gas flow. The residue was re-dissolved in a HPLC mobile phase, the solution was injected to HPLC device, and was assayed.

### HPLC conditions:

Mobile phase: acetonitrile/water/phosphoric acid =250/750/1
Flow rate: 0.5 mL/min
Column: CAPCELL PAK MF 4.0x 10 mm
Column temperature: 40°C
Detection: UV at 280 nm

**Table 5**

| | Aqueous injection | Suspension of ultrafine drug particles (172.7 nm) |
|---|---|---|
| Area under the plasma drug concentration (µg·hr/mL) | 33.67 ± 5.23 | 35.83 ± 6.59 |
| Maximum drug concentration in plasma (µg/mL) | 5.58 ± 0.85 | 6.59 ± 0.61 |

Fig. 7 and Table 5 show that the suspended injection of fine particles of N-(3-chloro-7-indolyl)-1,4-benzenedisulfonamide has disposition properties equivalent to those of the aqueous injection. These results demonstrate that fine drug particles having excellent disposition characteristics in intravascular administration equivalent to that of the aqueous solution can be produced according to the present invention.

### Examples

The present invention will be illustrated in further detail with reference to the following Examples which by no means limit the scope of the present invention.

### Example 1

In a Microfluidizer (Micorofluidics Inc.) was circulated 7 mL of a 1% aqueous solution of Pluronic F68. To the circulating solution was added 100 µL of a 50 mg/mL solution of N-(3-chloro-7-indolyl)-1,4-benzenedisulfonamide in acetone. The mixture was emulsified by pressurizing at 3000 psi for 20 minutes and thereby yielded fine particles of N-(3-chloro-7-indolyl)-1,4-benzenedisulfonamide having an average particle size of 218 nm.

### Example 2

A suspension of fine particles of N-(3-chloro-7-indolyl)-1,4-benzenedisulfonamide having an average particle size of 218 nm was prepared by circulating 7 mL of a 1% aqueous solution of Pluronic F68 in a Microfluidizer; adding 100 µL of a 50 mg/mL solution of N-(3-chloro-7-indolyl)-1,4-benzenedisulfonamide in acetone to the circulating solution; and pressurizing the mixture at 3000 psi for 20 minutes. The average particle size of fine drug particles in the resulting suspension was sequentially determined until 48 hours after the production, but no significant change was observed.

### Examples 3 to 5

A series of fine drug particles having average particle sizes of 172.7 nm, 178.8 nm, and 211.3 nm, respectively, were prepared by circulating 7 mL of a 1% aqueous solution of Pluronic F68 in a Nanomiser; adding 100 µL of a 50 mg/mL solution of N-(3-chloro-7-indolyl)-1,4-benzenedisulfonamide in acetone to the circulating solution; and pressurizing and emulsifying the mixture at 8700 psi (Example 3), 15600 psi (Example 4), and 20900 psi (Example 5), respectively, for 20 minutes.

### Examples 6 and 7

Fine drug particles of N-(3-chloro-7-indolyl)-1,4-benzenedisulfonamide were prepared by circulating 7 mL of a 1% aqueous solution of Pluronic F68 through a Nanomiser whose temperature in the emulsifier section was controlled to 5°C (Example 6) or 20°C (Example 7) using a constant-temperature water bath was circulated; adding 100 µL of a 50 mg/mL solution of N-(3-chloro-7-indolyl)-1,4-benzenedisulfonamide in acetone to the circulating solution; and carrying out 50-pass treatment (online treatment with a high-pressure homogenizer 50 times) of pressurization and emulsification at 18720 psi. The average particle size, clarity and color of the prepared fine drug particles were stable even after standing to cool for one hour.

### Example 8

Fine particles of Danazol (17β-hydroxy-2,4,17α-pregnadien-20-yno[2,3-d]isoxazole, Sigma-Aldrich) having an average particle size of 272.2 nm was prepared by circulating 7 mL of a 1% aqueous solution of Pluronic F68 in a Microfluidizer (Micorofluidics Inc.); adding 100 µL of a 50 mg/mL solution of Danazol in acetone to the circulating solution; and pressurizing and emulsifying the mixture at 3000 psi for 20 minutes.

The stability with time in average particle size of the fine drug particles was determined until after 72 hours (after 3 days) to find that the average particle size was stable and was substantially free from change, as shown in Table 6.

**Table 6 Example 8 (treatment with Microfluidizer)**

| Time (hr) | immediately after preparation | 1 hr | 2 hr | 3 hr | 4 hr | 72 hr |
|---|---|---|---|---|---|---|
| Average particle size (nm) | 272.2 | 270.1 | 278.8 | 277.7 | 280.5 | 276.7 |

### Example 9

A series of fine drug particles of N-(3-chloro-7-indolyl)-1,4-benzenedisulfonamide having average particle sizes of 187.4 to 324.0 nm were prepared by circulating 7 mL of a 1% aqueous solution of Pluronic F68 through a Nanomiser whose temperature in the emulsifier section was controlled to 5°C using a constant-temperature water bath; adding 100 µL of a 50 mg/mL solution of N-(3-chloro-7-indolyl)-1,4-benzenedisulfonamide in acetone to the circulating solution; pressurizing and emulsifying the mixture at 20350 psi; and carrying out 1, 3, 5, 7, 10, 20, 50, or 100-pass (times) online Nanomiser treatment and filtration through a 0.45-µm filter. The average particle sizes of the produced fine drug particles were stable and were substantially free from change immediately after production, 1 day later, and 7 days later, as shown in Table 7.

**Table 7 Example 9**

| Number of treatments (times) | Recovery of the filtration through 0.45µm (%) | Average particle size (nm) | | |
|---|---|---|---|---|
| | | immediately after production | 1 day later | 7 days later |
| 1 | 4.3 | | | |
| 3 | 3.3 | 324.0 | 336.4 | 334.7 |
| 5 | 5.0 | 295.1 | 298.2 | 307.9 |
| 7 | 8.5 | 356.1 | 355.7 | 368.3 |
| 10 | 9.9 | 253.4 | 257.9 | 262.8 |
| 20 | 101.3 | 216.0 | 218.4 | 224.1 |
| 50 | 103.7 | 192.7 | 201.6 | 233.1 |
| 100 | 80.0 | 187.4 | 193.1 | 215.7 |

The "number of treatments" in Table 7 means the number of times of Nanomiser treatments in which a sample was treated with a Nanomiser, the recovered mixture was immediately re-treated with the Nanomiser, and these procedures were repeated.

## Claims

1. A method of producing ultrafine drug particles having an average particle size of 10 nm to 1000 nm, comprising the steps of 1) dissolving a drug in at least one good solvent or a mixture of good solvents to prepare a drug-containing solution; 2) mixing the drug-containing solution with a solvent being a poor solvent or a mixture of poor solvents for the drug and being miscible with the drug-containing solution in the good solvent or a mixture of good solvents; and 3) subjecting the prepared mixture directly to emulsification under a set processing pressure using a high-pressure homogenizer, without carrying out any pretreatment step for adjusting the drug to have an average particle size of 100 µm or less.

2. The production method according to Claim 1, further comprising the steps of circulating the solvent being a poor solvent or a mixture of poor solvents for the drug and being miscible with the drug-containing solution in the good solvent or a mixture of good solvents through a channel in the high-pressure homogenizer, and adding the drug-containing solution to the circulating miscible solvent to thereby mix them.

3. The production method according to Claim 1 or 2,
wherein the drug is an insoluble drug having a solubility in water of 1 mg/ml or less.

4. The production method according to any one of Claims 1 to 3, further comprising dissolving a dispersing agent in a solvent of at least one of 1) the drug-containing solution in a good solvent or a mixture of good solvents and 2) the solvent being a poor solvent or a mixture of poor solvents for the drug and being miscible with the drug-containing solution in the good solvent or a mixture of good solvents.

5. The production method according to Claim 4,
wherein a concentration of the dispersing agent in the solvent in which the dispersing agent is dissolved is 0.01% to 50% (W/V).

6. The production method according to Claim 4 or 5,
wherein the dispersing agent is polyoxyethylene polyoxypropylene glycol, lecithin, gelatin and/or polyvinylpyrrolidone.

7. The production method according to any one of Claims 1 to 6, wherein, in the step of mixing the drug-containing solution with a solvent being a poor solvent or a mixture of poor solvents for the drug and being miscible with the drug-containing solution in the good solvent or a mixture of good solvents, the amount of the drug-containing solution is 0.01% to 50% (V/V) to the amount of the solvent being a poor solvent or a mixture of poor solvents for the drug and being.miscible with the drug-containing solution.

8. The production method according to any one of Claims 1 to 7, wherein the average particle size is 100 nm to 400 nm.

9. The production method according to any one of Claims 1 to 8, wherein the high-pressure homogenizer is a Microfluidizer, a piston-gap homogenizer, a Manton Gaulin Homogenizer, or a Nanomiser.

10. The production method according to Claim 9,
wherein the high-pressure homogenizer is a Microfluidizer or a Nanomiser.

11. The production method according to any one of Claims 1 to 10, wherein the drug is one of antitumor drugs, antibiotics, anti-inflammatory drugs, analgesics, drugs for treating osteoporosis, hypolipidemic drugs, antibacterial drugs, sedative drugs, tranquilizers, antiepileptic drugs, antidepressants, drugs for treating digestive system diseases, drugs for treating allergic diseases, antihypertensive drugs, antiarteriosclerosis drugs, antidiabetic drugs, hormone drugs and lipid soluble vitamin preparations.

12. The production method according to any one of Claims 1 to 11, wherein the high-pressure homogenizer is used at a set processing pressure of 500 to 40000 psi.

13. The production method according to Claim 12,
wherein the high-pressure homogenizer is a Microfluidizer and wherein the set processing pressure is 1000 to 6000 psi.

14. The production method according to Claim 12,
wherein the high-pressure homogenizer is a Nanomiser and
wherein the set processing pressure is 6000 to 20000 psi.

15. A method of producing a suspension of ultrafine drug particles or powdered ultrafine drug particles in an arbitrary concentration, the ultrafine drug particles having an average particle size of 10 nm to 1000 nm, comprising the steps of 1) dissolving a drug in a good solvent or a mixture of good solvents to prepare a drug-containing solution; 2) mixing the drug-containing solution with a solvent being a poor solvent or a mixture of poor solvents for the drug and being miscible with the drug-containing solution in the good solvent or a mixture of good solvents; 3) subjecting the prepared mixture directly to emulsification under a set processing pressure using a high-pressure homogenizer without carrying out a pretreatment step for adjusting the drug to have an average particle size of 100 µm or less; and 4) removing part or all of the solvent from the suspension of ultrafine drug particles after the treatment with the high-pressure homogenizer.

16. The production method according to Claim 15, further comprising the steps of circulating the solvent being a poor solvent or a mixture of poor solvents for the drug and being miscible with the drug-containing solution in the good solvent or a mixture of good solvents through a channel in the high-pressure homogenizer, and adding the drug-containing solution to the circulating miscible solvent to thereby mix them.

17. The production method according to one of Claims 15 and 16, wherein the step of removing part or all of the solvent from the suspension of ultrafine drug particles after the treatment with the high-pressure homogenizer is freeze-drying.

18. A high-pressure homogenizer equipped with an online injector, comprising a high-pressure homogenizer and an injector, the high-pressure homogenizer shown in the following Fig. 1 comprising a reservoir, a booster pump and an emulsifier, being connected via thin tubes, the injector being so configured as to feed a drug-containing solution containing a drug dissolved in a good solvent or a mixture of good solvents, the injector being integrated into the high-pressure homogenizer at any position of a channel for a circulating fluid in the thin tubes extending from the reservoir to the emulsifier.

19. The high-pressure homogenizer equipped with an online injector according to Claim 18, wherein the injector is integrated at any position of a channel in the thin tube connecting between the reservoir and the booster pump as shown in the following Fig. 2.

20. The high-pressure homogenizer equipped with an online injector according to Claim 18, wherein the injector is integrated at any position of a channel in the thin tube connecting between the booster pump and the emulsifier as shown in the following Fig. 3.

21. The high-pressure homogenizer equipped with an online injector according to any one of Claims 18 to 20,
wherein the injector is integrated at any position of a channel in the thin tubes via a joint and/or a mixer.

22. The high-pressure homogenizer equipped with an online injector according to any one of Claims 18 to 21, further comprising a regulator for controlling the temperature of the circulating fluid and/or the drug-containing solution, the regulator being integrated into part or all of the emulsifier and/or the thin tubes.

23. The production method according to Claim 2 or 15,
wherein the high-pressure homogenizer is the high-pressure homogenizer equipped with an online injector of any one of Claims 18 to 22.

24. A method of producing ultrafine drug particles having an average particle size of 10 nm to 1000 nm,
comprising the steps of 1) dissolving a drug in a good solvent or a mixture of good solvents to prepare a drug-containing solution; 2) circulating a solvent in a channel in the thin tubes of the high-pressure homogenizer equipped with an online injector of any one of Claims 18 to 22, the solvent being a poor solvent or a mixture of poor solvents for the drug and being miscible with the drug-containing solution in the good solvent or a mixture of good solvents; 3) feeding the drug-containing solution through the online injector to thereby mix the drug-containing solution with the circulating miscible solvent; and 4) directly emulsifying the resulting mixture online under a set processing pressure using the high-pressure homogenizer.

25. Use of the high-pressure homogenizer equipped with an online injector of Claim 18 for emulsification of a drug-containing solution.
